# EUROPEAN PATENT SPECIFICATION

(11) **EP 3 243 518 B1**
(45) Date of publication and mention of the grant of the patent: **10.04.2024**
(21) Application number: 17169931.7
(22) Date of filing: 08.05.2017
(51) Int. Cl.: A61K 35/54, A61K 38/24, A61P 15/00, A61P 15/08

(54) **FOLLICULAR FLUID FOR USE IN THE TREATMENT OF INFERTILITY**
FOLLIKULARES FLUID ZUR VERWENDUNG IN DER BEHANDLUNG VON UNFRUCHTBARKEIT
FLUIDE FOLLICULAIRE POUR UTILISATION DANS LE TRAITEMENT DE L' INFERTILITÉ

(30) Priority: 11.05.2016 IT UA20163349
(43) Date of publication of application: 15.11.2017
(73) Proprietor: Gionas S.r.l., 20121 Milano (IT)
(72) Inventor: MAGGIONI, Cristina, I-20121 MILANO (IT)
(74) Representative: Croce, Valeria

(56) References cited:
- EP-A1- 2 400 303
- WO-A1-2006/039604
- US-A1- 2001 049 829
- BIJTTEBIER J ET AL: "Preovulatory follicular fluid during in vitro maturation decreases polyspermic fertilization of cumulus-intact porcine oocytes", THERIOGENOLOGY, LOS ALTOS, CA, US, vol. 70, no. 4, 1 September 2008 (2008-09-01), pages 715-724, XP023314476, ISSN: 0093-691X, DOI: 10.1016/J.THERIOGENOLOGY.2008.04.046 [retrieved on 2008-06-24]
- MARTIN WILDING ET AL: "NAPLES, ITALY; The effect of extended culture of cumulus-oocyte complexes in follicular fluid during in vitro fertilisation cycles", JOURNAL OF ASSISTED REPRODUCTION AND GENETICS, KLUWER ACADEMIC PUBLISHERS-PLENUM PUBLISHERS, NE, vol. 23, no. 3, 19 April 2006 (2006-04-19) , pages 129-136, XP019398420, ISSN: 1573-7330, DOI: 10.1007/S10815-005-9016-Y

## Description

The present invention relates to follicular fluid for use in vitro fertilization, where said use comprises introducing follicular fluid in the uterus in one or more administrations during a time period comprised between six days before embryo transfer and the transfer day itself.

### Prior art

Among the assisted reproduction techniques, there is IVF-ICSI, which involves in vitro fertilization of the egg with a subsequent transfer of the embryo thus formed in the woman's uterus.

The procedure is essentially divided into four steps: controlled induction of ovulation; oocyte retrieval; in vitro fertilization; embryo transfer. Depending on whether in vitro fertilization takes place naturally or by intra cytoplasmic injection of gametes, the techniques are called IVF or ICSI, respectively.

Since the first IVF success in the late 70's to the present, the success rate of the technique has had no major improvements. The step of re-implantation of embryos in the uterus is the most important step, which could significantly influence the outcome of an IVF cycle.

The same author of the present document, in Martel D. et al. J Endocrinol. 1987 114(2) :319-24, noted that the moment in which the implantation of embryos occurs is, in humans, the fifth day after ovulation. However, in practice, the transfer of the blastocyst continues to be made on the second day after ovulation, unless material, oocytes or embryos, is used which has been stored from previous fertilizations.

The injection into the uterus before the blastocyst transfer of the supernatant of embryonic cultures has not improved the success rates of the implantation (Zhu W. et al. Fertil Steril. 2010 93(7):2216-21; Goto S. et al. Fertil Steril. 2009 92(4):1264-8).

On average, up to 90% of apparently healthy zygotes are destined to disappear when implanted in the uterus, without giving any sign of trophoblastic implantation and of production of human chorionic gonadotropin (hCG).

Since the different steps of IVF-ICSI are particularly challenging for women, both in physical and psychological terms, the need to increase the still disappointing success rates thereof is strongly felt. The implantation step is now the critical step on which to intervene with the aim of increasing the success rate of IVF-ICSI.

Follicular fluid is a combined product of the transfer of blood constituents that cross the follicular barrier and of the secretory activity of the granulose and cellular cells (Fortune J.E. Biol Reprod. 1994, 50: 225-232). The oocyte that is cyclically formed in the female reproductive system, either physiologically or typically in the context of assisted procreation procedures, following hormone induction, has a nucleus inside around which a single layer of flat cells is arranged, follicular cells. With the passing of days, the flat follicular cells become cubic and multiply, thus forming multiple concentric layers, until a space containing the follicular fluid is formed between them. As the maturation process progresses, the cortical layer surrounding the follicle proliferates, thus originating the theca interna and the theca externa. At full maturity, the theca interna disappears and the follicle, which protrudes at the surface of the ovary, breaks down. This rupture is accompanied by the escape of the follicular fluid and the expulsion of the mature oocyte.

The author of the present invention proposes an implantation method that has been shown to improve the success rate of IVF-ICSI, due to the improvements that the method surprisingly achieves in the critical implantation step.

### Description of the invention

The author of the present invention has surprisingly demonstrated that zygote implantations in the uterus have a greater implantation efficacy when said implantation is preceded by or occurs in conjunction with the introduction of follicular fluid in the uterus, wherein said follicular fluid is autologous follicular fluid, collected when picking the egg from the subject herself, or it is follicular fluid collected at the time of the oocyte explantation and stored frozen until the time of introduction into the uterus.

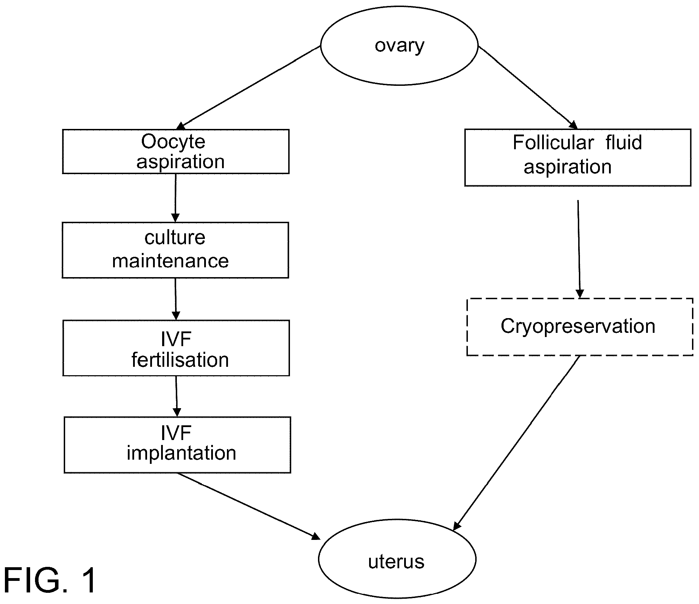

Figure 1 is a block diagram of the method according to the present invention.

An innovative method to be applied in the step of embryo transfer in the uterus is described but not claimed herein, where said method significantly increases the percentage of implantation in the uterus, described in detail in the following paragraphs and claims.

For the purposes of the present invention, the follicular fluid is obtained from the ovaries by any of the methods known to those skilled in the art. Typically, in an IVF or ICSI procedure, follicular fluid is readily available as it is aspirated along with the oocyte during the oocyte collection step.

For the purposes of the present invention, follicular fluid is a follicular fluid that has the characteristics of the follicular fluid just aspirated from the ovaries. In one embodiment, it is follicular fluid kept frozen, preferably at -80 °C, or in liquid nitrogen, from the aspiration to the time of use. Said follicular fluid is therefore a follicular fluid that comprises all components of the freshly aspirated follicular fluid unaltered, which does not undergo dilutions with other media or degradation due to inadequate storage conditions.

Figure 1 is a block diagram representing the innovative method described herein.

Said follicular fluid is aspirated when the oocytes that will be subjected to vitro fertilization are aspirated. The oocytes are then isolated by aspiration from the follicular fluid, after which said follicular fluid is introduced into the uterus or stored for subsequent use. To this end, said follicular fluid is stored for the introduction into the uterus at a later time. In both cases, follicular fluid is introduced into the uterus according to the definition given for the purposes of the present invention.

For the purposes of the present invention, the follicular fluid is preferably collected from the follicles in phase with the complete maturation of the oocyte, i.e. in the form in which they are found at the time of the fine needle aspiration.

The author of the present invention has surprisingly demonstrated that follicular fluid, introduced into the uterus, has an advantageous role in promoting the successful outcome of the embryo implantation. This effect is observed where said follicular fluid has all the characteristics of the freshly explanted follicular fluid. According to claim 1, said follicular fluid is introduced into the uterus in a volume comprised between 0.1 and 5 cm³, more preferably between 1.5 and 3 cm³, even more preferably in a volume of about 2 cm³.

Surprisingly, the authors of the present invention have shown that follicular fluid plays a crucial role in determining the temporal window of the endometrial implantation, where the presence of fresh follicular fluid, i.e. having the characteristics of the freshly aspirated follicular fluid, makes the maturation of the endometrium reach the optimum maturation stage for embryo implantation. For the purpose of the present invention, it is essential that said follicular fluid is properly stored follicular fluid, because after a few hours of aspiration, for example if maintained at room temperature, said follicular fluid undergoes a degradation of some of its fundamental components, a degradation which causes the follicular fluid to lose its surprising capacity shown herein to bring the endometrium to maturation. In a preferred embodiment, said fresh follicular fluid is not diluted with other culture media.

Follicular fluid is claimed herein for use in the treatment of infertility, characterized in that said follicular fluid is introduced into the uterus in one or more administrations during a time period comprised between six days before embryo transfer and the transfer day itself.

In a preferred embodiment, said use is in the embryo transfer process.

According to claim 2, said use is in improving the implantation success rate in a human by transfer of blastocysts, where follicular fluid is introduced into the uterine cavity of a human patient undergoing embryo transfer in one or more administrations during a time period comprised between six days before the embryo transfer and the day of the transfer itself.

In a further aspect, a pharmaceutical composition is described, comprising fresh follicular fluid for therapeutic use in the IVF-ICSI treatment and other extra-uterine fertilization procedures, in a volume comprised between 0.1 and 5 cm³, more preferably between 1.5 and 3 cm³, even more preferably about 2 cm³. According to claim 8, said composition is introduced into the uterus four, five or six days before the embryo transfer.

A method for producing a composition for use in improving the implantation success rate by transfer of blastocysts is not claimed, said method comprising the steps of collecting follicular fluid from the same subject in which said blastocyst transfer will be carried out, optionally storing it at -80°C or in liquid nitrogen, optionally admixing it with other active components. Said follicular fluid is preferably collected from follicles of at least 16 mm, or at least 20 mm.

A further aspect of the present invention is the follicular fluid for use in vitro fertilization, where said use comprises introducing follicular fluid in the uterus in one or more administrations during a time period comprised between six days before embryo transfer and the transfer day itself.

In a preferred embodiment, said follicular fluid is collected from mature follicles, preferably of at least 16 mm.

Even more preferably, said follicular fluid is autologous follicular fluid.

A further aspect of the present invention not claimed is a method for collecting and processing follicular fluid, comprising:
- collecting, preferably transvaginally, ovarian follicles from the ovary, by employing ultrasound techniques;
- puncturing the ovarian follicles and collecting the oocyte-containing follicular fluid;
- isolating the oocytes through aspiration;
- introducing the follicular fluid into the uterus or storing it.

According to claim 5, the combined intrauterine administration of a composition comprising the follicular fluid, or fractions thereof, together with one or more pharmacological agents, for example selected from progesterone, nitric oxide donors, aromatase inhibitors, ascorbic acid, glucocorticoids, insulin or insulin-sensitizing drugs, relaxin, HCG, thyroxine, melatonin, phytotherapeutic extracts, verbascum. The selection of said one or more pharmacological agents is carried out on the basis of specific patient's needs, as is well known to the man skilled in the art.

Alternatively, said introduction of the follicular fluid into the uterus is accompanied by the administration of one or more pharmacological agents that favor the uterine implantation. Said one or more pharmacological agents, selected from the group comprising, for example, progesterone, nitric oxide donors, aromatase inhibitors, ascorbic acid, glucocorticoids, insulin or insulin-sensitizing drugs, relaxin, HCG, thyroxine, melatonin, phytotherapeutic extracts, verbascum, is administered by an administration route selected, for example, from subcutaneously, intraperitoneally, intramuscularly, sublingually, orally. Said one or more agents are administered in one or more administrations within a time period comprised between fourteen days before the introduction of the follicular fluid into the uterus and up to 3 months after the introduction of the follicular fluid, or up to one month later, or up to fifteen days later, or up to five days later.

According to claim 9, a further object of the present invention is a pharmaceutical composition comprising: follicular fluid according to the definition given in the present invention, one or more pharmacological agents selected from the group comprising, for example, progesterone, nitric oxide donors, aromatase inhibitors, ascorbic acid, glucocorticoids, insulin or insulin-sensitizing drugs, relaxin, HCG, thyroxine, melatonin, phytotherapeutic extracts, verbascum, one or more pharmaceutically acceptable carriers.

Said composition finds application for use in in vitro fertilization, where said use comprises introducing said composition in the uterus in one or more administrations during a time period comprised between six days before embryo transfer and the transfer day itself.

In a further aspect, a method for IVF-ICSI and other extra-uterine fertilization procedures is described but not claimed herein, wherein said method comprises administering fresh autologous follicular fluid to a woman in one or more administrations, wherein said follicular fluid is introduced into the uterus in a volume of between 1 and 5 cm³, more preferably between 1.5 and 3 cm³, in a time period comprised between 6 days before the embryo transfer and the day of the transfer itself. Said method has surprisingly been shown to improve the implantation success rate.

Said method, described herein for humans, is advantageously applied also in other mammalian species.

## Claims

1. Fresh follicular fluid for use in the treatment of infertility, wherein said use comprises introducing said fresh follicular fluid into the uterus in one or more administrations during a time period comprised between 6 to 4 days before the embryo transfer in the uterus in a volume of 0,1 to 5 cm³, wherein said fresh follicular fluid is aspirated along with the oocyte and properly stored after aspiration, preferably at -80°C or in liquid nitrogen and it has been processed to isolate the oocyte contained therein.

2. Fresh follicular fluid for use according to claim 1, wherein said use is in improving the blastocyst implantation success rate.

3. Fresh follicular fluid for use according to any one of claims 1 or 2, wherein said follicular fluid is autologous follicular fluid.

4. Fresh follicular fluid for use according to any one of claims 1 to 3, wherein said follicular fluid is collected from mature follicles.

5. Fresh follicular fluid for use according to any one of claims 1 to 4, wherein said use is combined with the use of one or more pharmacological agents preferably selected from the group comprising progesterone, nitric oxide donors, aromatase inhibitors, ascorbic acid, glucocorticoids, insulin or insulin-sensitizing drugs, relaxin, HCG, thyroxine, melatonin, phytotherapeutic extracts, verbascum, and optionally one or more pharmaceutically acceptable carriers.

6. Fresh follicular fluid for use according to claim 5, wherein said one or more agents are administered by introduction into the uterus at the same time of introducing the follicular fluid into the uterus, or are administered in one or more administrations within a time period comprised between fourteen days before the introduction of the follicular fluid and up to 3 months after the introduction of the follicular fluid, or up to one month later, or up to fifteen days later, or up to five days later by an administration route selected from, for example, subcutaneously, intraperitoneally, intramuscularly, sublingually, orally.

7. Pharmaceutical composition comprising the fresh follicular fluid for use in the treatment of infertility according to claim 1, in a volume comprised between 0.1 and 5 cm³, more preferably between 1.5 and 3 cm³, even more preferably in a volume of about 2 cm³.

8. Pharmaceutical composition comprising the fresh follicular fluid for use in the treatment of infertility according to claim 7, wherein said composition is introduced into the uterus four, five or six days before the embryo transfer.

9. Pharmaceutical composition comprising the fresh follicular fluid for use in the treatment of infertility according to claim 7 or 8, further comprising one or more pharmacological agents, preferably selected from the group comprising progesterone, nitric oxide donors, aromatase inhibitors, ascorbic acid, glucocorticoids, insulin or insulin-sensitizing drugs, relaxin, HCG, thyroxine, melatonin, phytotherapeutic extracts, verbascum, one or more pharmaceutically acceptable carriers.

## Patentansprüche

1. Frisches follikulares Fluid zur Verwendung bei der Behandlung von Unfruchtbarkeit, wobei die Verwendung das Einbringen des frischen follikularen Fluids in den Uterus in einer oder mehreren Verabreichungen während eines Zeitraums zwischen 6 und 4 Tagen vor dem Embryotransfer in den Uterus in einem Volumen von 0,1 bis 5 cm³ umfasst, wobei das frische follikulare Fluid zusammen mit der Eizelle aspiriert und nach der Aspiration ordnungsgemäß gelagert wird, vorzugsweise bei -80°C oder in flüssigem Stickstoff, und es verarbeitet wurde, um die darin enthaltene Eizelle zu isolieren.

2. Frisches follikulares Fluid zur Verwendung nach Anspruch 1, wobei die Verwendung in der Verbesserung der Erfolgsrate der Blastozysten-Implantation liegt.

3. Frisches follikulares Fluid zur Verwendung nach einem der Ansprüche 1 oder 2, wobei das follikulare Fluid autologes follikulares Fluid ist.

4. Frisches follikulares Fluid zur Verwendung nach einem der Ansprüche 1 bis 3, wobei das follikulare Fluid aus reifen Follikeln gewonnen ist.

5. Frisches follikulares Fluid zur Verwendung nach einem der Ansprüche 1 bis 4, wobei die Verwendung mit der Verwendung eines oder mehrerer pharmakologischer Wirkstoffe kombiniert wird, die vorzugsweise aus der Gruppe ausgewählt sind, die Progesteron, Stickoxid-Donatoren, Aromatase-Inhibitoren, Ascorbinsäure, Glucocorticoide, Insulin oder Insulin-sensibilisierende Medikamente, Relaxin, HCG, Thyroxin, Melatonin, phytotherapeutische Extrakte, Verbascum und gegebenenfalls einen oder mehrere pharmazeutisch akzeptable Träger umfasst.

6. Frisches follikulares Fluid zur Verwendung nach Anspruch 5, wobei der eine oder die mehreren Wirkstoffe durch Einbringen in den Uterus zur gleichen Zeit wie das Einbringen des follikularen Fluids in den Uterus verabreicht werden, oder in einer oder mehreren Verabreichungen innerhalb eines Zeitraums zwischen vierzehn Tagen vor dem Einbringen des follikularen Fluids und bis zu drei Monaten nach dem Einbringen des follikularen Fluids verabreicht werden, oder bis zu einem Monat später oder bis zu fünfzehn Tage später oder bis zu fünf Tage später durch einen Verabreichungsweg, der beispielsweise aus subkutan, intraperitoneal, intramuskulär, sublingual oder oral ausgewählt ist.

7. Pharmazeutische Zusammensetzung, die das frische follikulare Fluid zur Verwendung bei der Behandlung von Unfruchtbarkeit nach Anspruch 1 umfasst, in einem Volumen zwischen 0,1 und 5 cm³, stärker bevorzugt zwischen 1,5 und 3 cm³, noch stärker bevorzugt in einem Volumen von etwa 2 cm³.

8. Pharmazeutische Zusammensetzung, die das frische follikulare Fluid zur Verwendung bei der Behandlung von Unfruchtbarkeit nach Anspruch 7 umfasst, wobei die Zusammensetzung vier, fünf oder sechs Tage vor dem Embryotransfer in den Uterus eingeführt wird.

9. Pharmazeutische Zusammensetzung, die das frische follikulare Fluid zur Verwendung bei der Behandlung von Unfruchtbarkeit nach Anspruch 7 oder 8 umfasst, ferner umfassend einen oder mehrere pharmakologische Wirkstoffe, die vorzugsweise aus der Gruppe ausgewählt sind, die Progesteron, Stickoxid-Donatoren, Aromatase-Inhibitoren, Ascorbinsäure, Glucocorticoide, Insulin oder Insulin-sensibilisierende Medikamente, Relaxin, HCG, Thyroxin, Melatonin, phytotherapeutische Extrakte, Verbascum und einen oder mehrere pharmazeutisch akzeptable Träger umfasst.

## Revendications

1. Fluide folliculaire frais pour utilisation dans le traitement de l'infertilité, dans lequel ladite utilisation comprend l'introduction dudit fluide folliculaire frais dans l'utérus en une ou plusieurs administrations pendant une période comprise entre 6 à 4 jours avant le transfert embryonnaire dans l'utérus selon un volume de 0,1 à 5 cm³, dans lequel ledit fluide folliculaire frais est aspiré conjointement avec l'ovocyte et stocké de façon adéquate après aspiration, de préférence à -80°C ou dans de l'azote liquide et a été traité pour isoler l'ovocyte contenu dans celui-ci.

2. Fluide folliculaire frais pour utilisation selon la revendication 1, dans lequel ladite utilisation vise à améliorer le taux de réussite d'implantation du blastocyste.

3. Fluide folliculaire frais pour utilisation selon l'une quelconque des revendications 1 ou 2, dans lequel ledit fluide folliculaire est un fluide folliculaire autologue.

4. Fluide folliculaire frais pour utilisation selon l'une quelconque des revendications 1 à 3, dans lequel ledit fluide folliculaire est collecté à partir de follicules matures.

5. Fluide folliculaire frais pour utilisation selon l'une quelconque des revendications 1 à 4, dans lequel ladite utilisation est combinée avec l'utilisation d'un ou plusieurs agents pharmacologiques de préférence sélectionnés dans le groupe comprenant la progestérone, des donneurs d'oxyde nitrique, des inhibiteurs d'aromatase, l'acide ascorbique, des glucocorticoïdes, l'insuline ou des médicaments sensibilisant à l'insuline, la relaxine, la HCG, la thyroxine, la mélatonine, des extraits phytothérapeutiques, le verbascum, et éventuellement un ou plusieurs vecteurs pharmaceutiquement acceptables.

6. Fluide folliculaire frais pour utilisation selon la revendication 5, dans lequel ledit ou lesdits agents sont administrés par introduction dans l'utérus en même temps que l'introduction du fluide folliculaire dans l'utérus, ou sont administrés en une ou plusieurs administrations sur une période comprise entre quatorze jours avant l'introduction du fluide folliculaire et jusqu'à 3 mois après l'introduction du fluide folliculaire, ou jusqu'à un mois plus tard, ou jusqu'à quinze jours plus tard, ou jusqu'à cinq jours plus tard par une voie d'administration sélectionnée parmi, par exemple, la voie sous-cutanée, la voie intrapéritonéale, la voie intramusculaire, la voie sublinguale, la voie orale.

7. Composition pharmaceutique comprenant le fluide folliculaire frais pour utilisation dans le traitement de l'infertilité selon la revendication 1, selon un volume compris entre 0,1 et 5 cm³, plus préférentiellement entre 1,5 et 3 cm³, encore plus préférentiellement en un volume d'environ 2 cm³.

8. Composition pharmaceutique comprenant le fluide folliculaire frais pour utilisation dans le traitement de l'infertilité selon la revendication 7, dans laquelle ladite composition est introduite dans l'utérus quatre, cinq ou six jours avant le transfert embryonnaire.

9. Composition pharmaceutique comprenant le fluide folliculaire frais pour utilisation dans le traitement de l'infertilité selon la revendication 7 ou 8, comprenant en outre un ou plusieurs agents pharmacologiques, de préférence sélectionnés dans le groupe comprenant la progestérone, des donneurs d'oxyde nitrique, des inhibiteurs d'aromatase, l'acide ascorbique, des glucocorticoïdes, l'insuline ou des médicaments sensibilisant à l'insuline, la relaxine, la HCG, la thyroxine, la mélatonine, des extraits phytothérapeutiques, le verbascum, et éventuellement un ou plusieurs vecteurs pharmaceutiquement acceptables.
